Europäisches Patentamt

European Patent Office

Office européen des brevets

(11) Veröffentlichungsnummer: **0 158 239**
**B1**

(12) **EUROPÄISCHE PATENTSCHRIFT**

(45) Veröffentlichungstag der Patentschrift :
19.10.88

(51) Int. Cl.⁴ : **C 07 C 69/533**, C 07 C 67/54

(21) Anmeldenummer : 85103771.3

(22) Anmeldetag : 28.03.85

(54) **Verfahren zur Gewinnung von Pentensäure C1-bis C4-Alkylestern.**

(30) Priorität : 10.04.84 DE 3413448

(43) Veröffentlichungstag der Anmeldung :
16.10.85 Patentblatt 85/42

(45) Bekanntmachung des Hinweises auf die Patenterteilung : 19.10.88 Patentblatt 88/42

(84) Benannte Vertragsstaaten :
BE CH DE FR GB IT LI NL

(56) Entgegenhaltungen :
EP-A- 0 126 349
FR-A- 2 356 622
US-A- 3 161 672

(73) Patentinhaber : BASF Aktiengesellschaft
Carl-Bosch-Strasse 38
D-6700 Ludwigshafen (DE)

(72) Erfinder : Schneider, Heinz-Walter, Dr.
Bruesseler Ring 43
D-6700 Ludwigshafen (DE)
Erfinder : Kummer, Rudolf, Dr.
Kreuzstrasse 6
D-6710 Frankenthal (DE)
Erfinder : Leman, Otto
Im Wandelgarten 8
D-6719 Weisenheim (DE)
Erfinder : Panitz, Paul
Wachenheimer Strasse 1
D-6520 Worms (DE)

## Beschreibung

Gegenstand der Erfindung ist ein Verfahren zur Gewinnung von Pentensäure C₁- bis C₄-Alkylestern aus solche enthaltenden Reaktionsgemischen, die durch Umsetzen von Butadien oder Butadien enthaltenden Kohlenwasserstoffgemischen mit Kohlenmonoxid und C₁- bis C₄-Alkanolen in Gegenwart von Cobaltcarbonylkatalysatoren und tert. Stickstoffbasen bei erhöhter Temperatur und unter erhöhtem Druck erhalten worden sind durch Destillation.

Die Herstellung von Pentensäureestern durch Umsetzen von Butadien oder solches enthaltenden Kohlenwasserstoffgemischen mit Kohlenmonoxid und Alkanolen in Gegenwart von Kobaltcarbonylkatalysatoren und tert. Stickstoffbasen ist aus der GB-PS 1 578 797 bekannt. Aus dem Reaktionsgemisch werden die Pentensäurealkylester durch Destillation abgetrennt. Es hat sich jedoch herausgestellt, daß sich bei der Destillation ein Teil des Katalysators zersetzt, während ein anderer Teil des Katalysators mit den Pentensäurealkylestern übergeht und diesen verunreinigt. Dies führt zu aufwendigen technischen Reinigungsoperationen und zudem zu Ablagerungen in den Vorrichtungen.

Es war deshalb die technische Aufgabe gestellt, Pentensäure C₁- bis C₄-Alkylester aus solche enthaltenden Reaktionsgemischen abzutrennen, wobei die so gewonnene Pentensäure C₁- bis C₄-Alkylester nicht durch Cobaltcarbonylkatalysatoren verunreinigt sind, sich bei der Destillation die Cobaltcarbonylkatalysatoren nicht zersetzen und in einer wieder verwendbaren Form anfallen.

Diese technische Aufgabe wird gelöst in einem Verfahren zur Gewinnung von Pentensäure C₁- bis C₄-Alkylestern aus solche enthaltenden Reaktionsgemischen, die durch Umsetzen von Butadien oder Butadien enthaltenden Kohlenwasserstoffgemischen mit Kohlenmonoxid und C₁- bis C₄-Alkanolen in Gegenwart von Cobaltcarbonylkatalysatoren und tert. Stickstoffbasen bei erhöhter Temperatur und unter erhöhtem Druck erhalten worden sind durch Destillation, wobei man

a) das von überschüssigem Kohlenmonoxid befreite flüssige Reaktionsgemisch bei erhöhter Temperatur unter einem Druck von 5 bis 50 bar mit Wasserstoff behandelt

b) aus dem so erhaltenen flüssigen Reaktionsgemisch Kohlenwasserstoffe abdestilliert

c) anschließend unter vermindertem Druck Pentensäure C₁- bis C₄-Alkylester, Alkanole und tert. Stickstoffbasen abdestilliert und

d) aus dem Pentensäure C₁- bis C₄-Alkylester, Alkanole und tert. Stickstoffbasen enthaltenden Destillat Pentensäure C₁- bis C₄-Alkylester durch fraktionierte Destillation gewinnt.

Das neue Verfahren hat den Vorteil, daß man Pentensäure C₁- bis C₄-Alkylester frei von Cobaltcarbonylverbindungen gewinnt, die Zersetzung von Cobaltcarbonylkatalysatoren vermeidet und die Cobaltcarbonylkatalysatoren in einer wieder verwendbaren aktiven Form erhält.

Bei der Herstellung von Pentensäure C₁- bis C₄-Alkylestern geht man von reinem Butadien-1.3 oder Butadien enthaltenden Kohlenwasserstoffgemischen aus. Solche Kohlenwasserstoffgemische enthalten z. B. neben Butadien gesättigte Kohlenwasserstoffe mit 3 bis 5 Kohlenstoffatomen und einfach olefinisch ungesättigte Kohlenwasserstoffe mit 3 bis 5 Kohlenstoffatomen. Der Butadiengehalt soll in der Regel mehr als 10 Gew.% betragen. In der Technik werden insbesondere C₄-Schnitte als Ausgangsgemisch verwendet. Als solches seien alle Gemische von übewiegend unverzweigten C₄-Kohlenwasserstoffen definiert die mehr als 10 Gew.% Butadien-1.3 enthalter. Typische Gemische enthalten 40 bis 60 Gew.% Butadien, 20 bis 35 Gew.% Isobuten, 10 bis 25 Gew.% Buten-1,5 bis 15 Gew.% Buten-2 und 1 bis 10 Gew.% Butane. Solche C₄-Schnitte fallen beispielsweise an bei der Dehydrierung von Butan oder Buten sowie als Nebenprodukte bei der Ethylengewinnung durch thermische Spaltung und Leichtbenzin oder höheren Kohlenwasserstoffschnitten.

Die Umsetzung erfolgt mit C₁- bis C₄-Alkanolen, insbesondere Methanol im Überschuß, z. B. von 1,5 bis 5 Mol, C₁- bis C₄-Alkanole je Mol Butadien.

Die Umsetzung wird bei erhöhter Temperatur und unter erhöhtem Druck durchgeführt. Vorzugsweise führt man die Umsetzung bei einer Temperatur von 80 bis 150 °C, insbesondere von 120 bis 140 °C und unter Drücken von 300 bis 1 200 bar, insbesondere 600 bis 1 200 bar durch. Kohlenmonoxid wird vorteilhaft im Überschuß, z. B. dem 1,5- bis 10-fachen der stöchiometrisch erforderlichen Menge angewandt.

Die verwendeten Cobaltkatalysatoren bringt man vorteilhaft bereits als Cobaltcarbonyl, insbesondere gelöst in Butadien oder C₄-Schnitt in das Gemisch ein. Besonders vorteilhaft verwendet man die bei der Reaktion wiedergewonnenen Cobaltcarbonylkatalysatoren. Es hat sich bewährt, wenn man 0,05 bis 0,15 g Atom Cobalt in Form von Cobaltcarbonyl je Mol Butadien anwendet.

Die mitverwendeten tert. Stickstoffbasen haben vorteilhaft einen $PK_a$-Wert von 3 bis 11. Vorzugsweise verwendet man N-heterocyclische Verbindungen wie Pyridin, Methylpyridine wie 3-Picolin, Isochinolin, Chinolin oder deren Gemische. Besondere technische Bedeutung hat Pyridin erlangt. Vorteilhaft wendet man 0,5 bis 1,5 Mol, insbesondere 0,8 bis 1,2 Mol tert. Stickstoffbasen je Mol Butadien an.

Nach beendeter Umsetzung wird das überschüssige Kohlenmonoxid vom flüssigen Reaktionsgemisch durch Entspannen abgetrennt. Das so erhaltene flüssige Reaktionsgemisch enthält neben nicht umgesetzten Butadien gegebenenfalls Kohlenwasserstoffe, tert. Stickstoffbasen, Cobaltcarbonylkatalysatoren, überschüssige Alkanole den als Wertprodukt erzeugten Pentensäurealkylester sowie Nebenprodukte wie Valeriansäureester, Butylketone sowie Polymerisate des Butadiens. Geeignete Gemische erhält man z. B. nach dem in der GB-PS 1 578 797 beschriebenen Verfahren.

Das flüssige Reaktionsgemisch wird bei erhöhter Temperatur, vorzugsweise von 80 bis 130 °C, insbesondere von 100 bis 120 °C unter einem Druck von 5 bis 80 bar, vorteilhaft 10 bis 50 bar mit Wasserstoff behandelt. Die Behandlungsdauer beträgt vorteilhaft 1 bis 60 Minuten.

Aus dem so erhaltenen flüssigen Reaktionsgemisch werden zweckmäßig nach Abtrennen des Wasserstoffs die darin enthaltenen Kohlenwasserstoffe abdestilliert. Anschließend wird unter vermindertem Druck z. B. 1 bis 200 mbar Pentensäure $C_1$- bis $C_4$-Alkylesteralkanole und Stickstoffbasen sowie ggf. Nebenprodukte abdestilliert. Vorteilhaft hält man bei der Destillation eine Sumpftemperatur < 80 °C, insbesondere von 40 bis 70 °C ein. Der im Destillationssumpf zurückbleibende Cobaltcarbonylkatalysator behält seine Aktivität und kann wieder für die Umsetzung eingesetzt werden.

Aus dem Pentensäure-$C_1$- bis $C_4$-Alkylester Alkanole und tert.-Stickstoffbasen sowie ggf. Nebenprodukte enthaltenden Destillat wird Pentensäure-$C_1$- bis $C_4$-Alkylester durch fraktionierte Destillation gewonnen.

Pentensäure $C_1$- bis $C_4$-Alkylester, die nach der Erfindung erhältlich sind, eignen sich zur Herstellung von Adipinsäureestern oder von δ-Formylvaleriansäurealkylestern, einem Vorprodukt zur Herstellung von Caprolactam.

Das Verfahren nach der Erfindung sei an folgendem Beispiel veranschaulicht.

### Beispiel

Ein Hochdruckgefäß von 2 l Inhalt wird stündlich mit 450 ml $C_4$-Schnitt, der 40 Gew.% (2 Mol) Butadien und 0,8 g Cobalt als Cobaltcarbonylwasserstoff enthält, 100 ml (2 Mol) Pyridin, 96 ml (2,4 Mol) Methanol und 32 ml Katalysatorlösung mit 2,4 g Cobaltcarbonyl aus dem Destillationssumpf beschickt. Gleichzeitig werden 160 N l Kohlenmonoxid zugeführt. Die Carbonylierung läuft bei 135 °C und 900 bar ab. Das aus dem Hochdruckgefäß abgenommene Produkt wird entspannt, wobei überschüssiges Kohlenmonoxid abgetrennt wird. Danach wird der flüssige Reaktoraustrag kontinuierlich bei 110 °C und 30 bar Wasserstoffdruck mit einer Verweilzeit von 10 Minuten mit Wasserstoff behandelt. Nach dem Entspannen werden zunächst die nicht umgesetzten $C_4$-Kohlenwasserstoffe bei Normaldruck und einer Sumpftemperatur von 90 °C abdestilliert. Danach trennt man unter vermindertem Druck (50 mbar und 60 °C) Pentensäuremethylester, Methanol und Pyridin sowie Nebenprodukte ab. Als Sumpf in der Kolonne fallen stündlich 40 ml Katalysatorlösung mit 3,2 g Kobalt an. Am Kopf der Kolonne wird ein cobaltfreies Gemisch aus nicht umgesetztem Methanol, Pyridin und Pentensäuremethylester abgezogen. Im Sumpf der Kolonne kann unter den eingestellten Bedingungen keine Cobaltabscheidung beobachtet werden. Das im Kopf der Kolonne abgenommene Destillat wird in einer weiteren Kolonne in Methanol, Pyridin und Pentensäuremethylester aufgetrennt. Diese Destillation wird unter Normdruck durchgeführt. Dabei werden stündliche am Kopf der Kolonne 16 ml Methanol und 160 ml Pyridin entnommen und in die Umsetzung zurückgeführt. Im Sumpf der Kolonne werden stündlich 225 ml Pentensäuremethylester (92 % Ausbeute) erhalten.

Der Cobaltcarbonyl-Katalysator enthaltende Sumpf der Kolonne wird nun üblicherweise geteilt. Etwa 80 % gehen in die Synthese zurück, 20 % werden ausgeschleust und oxidativ aufgearbeitet, wobei der Cobaltkatalysator als $Co^{2+}$-Salz zurückgewonnen wird.

### Patentansprüche

1. Verfahren zur Gewinnung von Pentensäure $C_1$- bis $C_4$-Alkylestern aus solche enthaltenden Reaktionsgemischen, die durch Umsetzen von Butadien oder Butadien enthaltenden Kohlenwasserstoffgemischen mit Kohlenmonoxid und $C_1$- bis $C_4$-Alkanolen in Gegenwart von Cobaltcarbonylkatalysatoren und tertiären Stickstoffbasen bei erhöhter Temperatur und unter erhöhtem Druck erhalten worden sind, durch Destillation, dadurch gekennzeichnet, daß man

a) das von überschüssigem Kohlenmonoxid befreite flüssige Reaktionsgemisch bei einer Temperatur von 80 bis 130 °C und unter einem Druck von 5 bis 80 bar mit Wasserstoff behandelt

b) aus dem so erhaltenen flüssigen Reaktionsgemisch Kohlenwasserstoffe abdestilliert

c) anschließend unter vermindertem Druck Pentensäure $C_1$- bis $C_4$-Alkylester, Alkanole und tert.-Stickstoffbasen abdestilliert und

d) aus dem Pentensäure $C_1$- bis $C_4$-Alkylester, Alkanole und tert. Stickstoffbasen enthaltenden Destillat Pentensäure $C_1$- bis $C_4$-Alkylester durch fraktionierte Destillation gewinnt.

2. Verfahren nach Anspruch 1, dadurch gekennzeichnet, daß man die Behandlung mit Wasserstoff unter einem Druck von 10 bis 50 bar durchführt.

3. Verfahren nach den Ansprüchen 1 und 2, dadurch gekennzeichnet, daß man Pentensäure $C_1$- bis $C_4$-Alkylester, Alkanole oder tert. Stickstoffbasen bei einer Temperatur kleiner 80 °C, aus dem Kobaltcarbonylkatalysatoren enthaltenden Reaktionsgemisch abdestilliert.

### Claims

1. A process for obtaining a $C_1$-$C_4$-alkyl pentenoate, by distillation, from a reaction mixture which contains this and which is obtained by reacting butadiene, or a butadiene-containing hydrocarbon mixture, with carbon monoxide and a $C_1$-$C_4$-alkanol in the presence of a cobalt carbonyl catalyst and a tertiary nitrogen base at elevated temperatures and under superatmospheric pressure, wherein

a) the liquid reaction mixture freed from excess carbon monoxide is treated with hydrogen at from 80 to 130 °C and under from 5 to 80 bar,

b) hydrocarbons are distilled off from the resulting liquid reaction mixture,

c) the $C_1$-$C_4$-alkyl pentenoate, the alkanol and the tertiary nitrogen base are then distilled off under reduced pressure, and

d) the $C_1$-$C_4$-alkyl pentenoate is obtained by fractional distillation from the distillate containing this compound, the alkanol and the tertiary nitrogen base.

2. A process as claimed in claim 1, wherein the treatment with hydrogen is carried out under from 10 to 50 bar.

3. A process as claimed in claim 1, wherein the $C_1$-$C_4$-alkyl pentenoate, the alkanol and the tertiary nitrogen base are distilled off at below 80 °C from the reaction mixture containing the cobalt carbonyl catalyst.

**Revendications**

1. Procédé d'obtention par distillation d'esters alkyliques en $C_1$-$C_4$ d'acide penténique, à partir de mélanges de réaction qui en contiennent et qui ont été obtenus par réaction de butadiène ou de mélanges d'hydrocarbures contenant du butadiène, avec de l'oxyde de carbone et des alcanols en $C_1$ à $C_4$, en présence de catalyseurs cobalt-carbonyle et de bases azotées tertiaires à température élevée et sous pression élevée, caractérisé par le fait que

a) on traite à l'hydrogène le mélange de réaction liquide débarrassé de l'oxyde d'azote en excès, à une température de 80 à 130 °C et sous une pression de 5 à 80 bar

b) on sépare par distillation les hydrocarbures du mélange de réaction liquide ainsi obtenu

c) puis on sépare par distillation, sous pression réduite, les esters alkyliques en $C_1$ à $C_4$ d'acide penténique, alcanols et bases azotées tertiaires, et

d) du distillat contenant les esters alkyliques en $C_1$ à $C_4$ d'acide penténique, alcanols et bases azotées tertiaires, on récupère, par distillation fractionnée, les esters alkyliques en $C_1$ à $C_4$ d'acide penténique.

2. Procédé selon la revendication 1, caractérisé par le fait que l'on effectue le traitement avec l'hydrogène sous une pression de 10 à 50 bar.

3. Procédé selon les revendications 1 et 2, caractérisé par le fait que l'on sépare, par distillation, du mélange de réaction contenant des catalyseurs cobalt-carbonyle, à une température inférieure à 80 °C, les esters alkyliques en $C_1$ à $C_4$ d'acide penténique, alcanols et bases azotées tertiaires.